# EUROPEAN PATENT SPECIFICATION

(11) **EP 2 508 896 B1**
(45) Date of publication and mention of the grant of the patent: **17.04.2019**
(21) Application number: 10834344.3
(22) Date of filing: 25.10.2010
(51) Int. Cl.: G01N 35/00, G01N 35/02

(54) **AUTOMATIC ANALYZING DEVICE**
AUTOMATISCHES ANALYSEGERÄT
DISPOSITIF D'ANALYSE AUTOMATIQUE

(30) Priority: 03.12.2009 JP 2009275044
(43) Date of publication of application: 10.10.2012
(73) Proprietor: Hitachi High-Technologies Corporation, Tokyo 105-8717 (JP)
(72) Inventor: KANEKO, Yasuo, Hitachinaka-shi Ibaraki 312-8504 (JP); KAMOSHIDA, Koji, Hitachinaka-shi Ibaraki 312-8504 (JP)
(74) Representative: Strehl Schübel-Hopf & Partner
(86) International application number: PCT/JP2010/006289
(87) International publication number: WO 2011/067888

(56) References cited:
- EP-A2- 1 760 471
- JP-A- 7 159 412
- JP-A- 10 300 755
- JP-A- 62 172 477
- JP-A- 2005 121 491
- JP-A- 2007 147 477
- JP-A- 2007 315 785
- JP-A- 2008 051 723
- US-A1- 2007 268 307

## Description

### Technical Field

The present invention relates to an automatic analyzing device that implements measurement for biogenic samples such as blood and urine. In particular, the present invention relates to an automatic analyzing device having a display screen that displays information relating to analysis, such as an analysis result.

### Background Art

When samples are analyzed by an automatic analyzing device, a bar code or the like in which identification information for uniquely identifying each of the samples is recorded is affixed on each sample container that contains a sample collected from a patient in advance. Request information for requesting examination is input based on the identification information acquired from an operating unit of an analyzing device or a host system connected through a network, and the bar code is read by a bar code reader or the like installed in the analyzing device to implement the requested analysis.

Further, since hundreds of kinds of reagents are present in recent years for use on analyzing devices to implement the analysis, from the viewpoint of quality control, bar codes or the like in which identification information for uniquely identifying the reagents, such as manufacturing information of reagents, is recorded, are affixed on containers, and managed and read by the bar code reader or the like installed in the analyzing device to register it therein for use in the analysis.

Automatic analyzing devices in recent years have become increasingly larger-sized and automated, and schemes are employed in which the device automatically reads, immediately before the analysis, identification information affixed on the sample containers and examination engineers are required only to introduce samples into the device. Also from the preventive standpoint against specimen misidentification, the necessity to recognize identification information immediately before the examination is one reason that motivates the above-stated scheme. Further, from the standpoint of physical arrangement and improvement in reading precision, reading means for reading identification information are fixed to an analyzing unit for implementing analysis. The same explanation as above applies also to examination reagents.

Accordingly, the identification information affixed on the containers of samples or reagents have had limited application as information to be read, at the time of introduction or the disposition to the device, for the identification of the content loaded or disposed on the device. Further, in the automatic analyzing devices, increase in the size, rise in the throughput and the integration of different kinds of examinations are advancing, and an automatic analyzing device has come into the mainstream that is called a module assembly scheme in which a plurality of analyzing units that implement examination are jointed and one network-connected operating unit manages them in an integrated manner.

An automatic analyzing device of this kind is described in, for example, Patent Literature 1.

### Citation List

### Patent Literature

Patent Literature 1: Japanese Patent Laid-Open Publication No. 9-281113

EP 1 760 471 A2 discloses an automatic ananlyzer with the features in the preamble of claim 1.

### Summary of Invention

### Technical Problem

In recent years, from rising consciousness for guaranteeing analysis results, automatic analyzing devices have come to have increased numbers of functions related to calibration and accuracy control, other than basic patient samples analysis. Further, the devices are experiencing advancement for systematization to operate in conjunction with a higher-order system or a remote system by using a network, departing from a mere solid-state device. Therefore, the number of screens needed for operating the automatic analyzing device is increasing and the structure continues to become more complex. Therefore, users needed to understand many screens and complex screen structures in order to implement necessary analysis and confirmation of the analysis result.

Further, the identification information of the samples to be used in the automatic analyzing device needs to uniquely identify the samples. Therefore, in facilities such as a large hospital, the identification information needs to be comprised of several tens of digits. Similarly, identification information for reagents used in the analysis also have several tens of digits for identification of the kind of reagents, production lot and packing unit. Further, in a large hospital, the number of results of analysis counts up to hundreds to several thousands. Therefore, it is extremely difficult to visually confirm the identification information displayed on the screen one by one.

Further, in conventional automatic analyzing devices, searching functions prepared for a part of screens are of a type in which the users manually input the identification information, in which there is a criticality involving material risks of mistakenly selecting a specimen, due to man-caused mistakes that might take place in copying information. Moreover, ranges of searching have been also simply configured such that searches are within a screen being displayed.

However, identification information of samples and reagents is information used commonly on the many kinds of screens including analysis request, analysis result, reagents management, calibration, and precision control; and is key information that is important on the analysis. In order to acquire all the information related thereto, users have spent enormous effort, such as, searching all the related screens. In particular, in laboratories assumed to be the usage conditions of the device, there are immeasurable efforts spent by users including night workers or doctors who do not usually use the automatic analyzing device and unfamiliar thereto but implement the same search as stated above.

An object of the present invention is to provide an automatic analyzing device having a function to mechanically read identification information affixed on the containers of the samples and reagents used in the analysis and transversally and automatically searching for referable test information from the test information stored in the automatic analyzing device and displayed on the screen and performing display output to guide users to a detailed screen, and thereby it is possible to improve work efficiency, exclude human mistakes and improve analysis efficiency.

### Solution to Problem

In order to achieve the above-described object, the present invention provides the automatic ananlyzer device as defined in claim 1. It comprises reading means for reading identification information of samples and reagents, such as, a bar code reader or an RFID reader in an operating unit that manages a single or a plurality of analyzing units that implement analysis in an integrated manner and a function to cause the reading means to read the identification information affixed on the containers of reagents or samples to thereby automatically search for the test information referable from the test information stored in the test information storage means in the operating unit and display the test information as a search result on the screen of the operating unit.

Further, the device has a function to allow the user to select target test information to thereby switch from a search result displayed on the screen to a detailed screen that displays details of the test information and a function to return to a search result from the detailed screen.

### Advantageous Effects of Invention

According to the present invention, it is possible to provide an analyzing device that can eliminate understanding of complex structures of screens, operation in a screen and manual input from a terminal to thereby improve work efficiency and exclude man-caused mistakes by only mechanically identification information to be read affixed on the containers of the samples or the examination reagents by identification information reading means, automatically search for and refer to the request-for- examination information for the sample and the test result, setting regarding the reagent, and result of analysis in which the reagents are used. Furthermore, by performing an analysis request and various setting operations by utilizing the present invention after switching to a target screen, it is possible to expect an effect to be utilized as an analysis operation aid function, not merely used as information reference. Furthermore, it is possible to suppress a device cost by providing the identification information reading means for the samples and reagents for the purpose of the information reference only for an operating unit for managing in an integrated manner a single or a plurality of analyzing units that implements analysis.

### Brief Description of Drawings

FIG. 1 is a diagram showing a relationship between a work flow in which the automatic analyzing device of the present invention is used and screen switching.
FIG. 2 is an exemplary system configuration of the automatic analyzing device of the present invention.
FIG. 3 shows an example of a test information search menu screen.
FIG. 4 shows a flowchart of searching process of test information.
FIG. 5 is a matrix showing correspondence between identification information to be read and within-the-search-coverage screen.
FIG. 6 shows an example of a search coverage setting screen.
FIG. 7 shows an example of a search result screen.
FIG. 8 shows an example of a test information detailed screen that is to be a reference target.
FIG. 9 shows a flowchart of an analysis implementability determination process.
FIG. 10 shows an example of an analysis implementability confirmation screen.

### Description of Embodiments

Hereafter, embodiments of the present invention will be described based on the accompanying drawings.

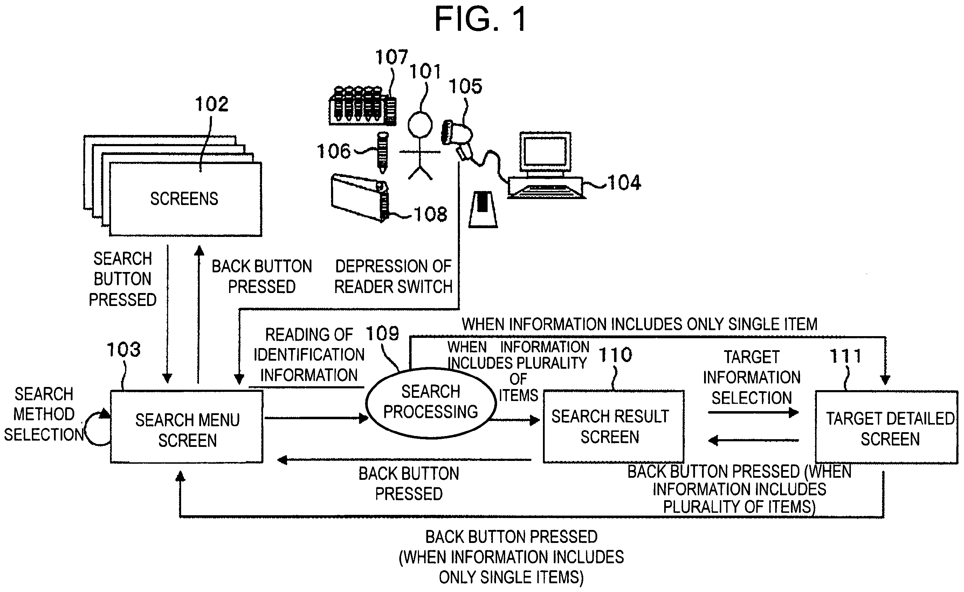

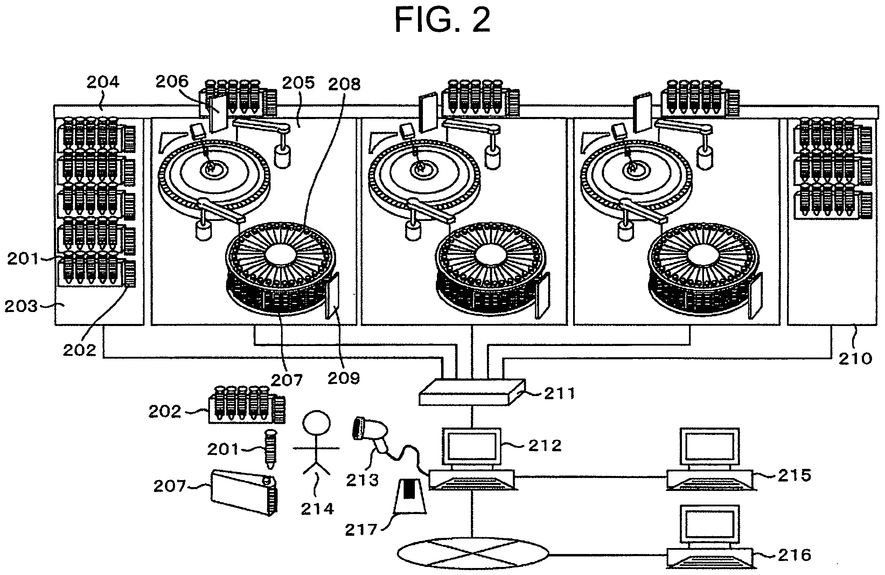

FIG. 2 is a system configuration diagram that shows an embodiment of the automatic analyzing device of the present invention. On each container of the sample to be analyzed, a sample ID 201 that uniquely identifies each sample is affixed, and the sample containers are loaded on a sample rack that can be loaded with a plurality of samples. On the sample rack, a sample rack ID 202 that uniquely identifies each sample rack is affixed. With the samples being loaded thereon, the sample racks are installed on the sample loading unit 203, and the installed sample racks are sequentially carried in the transportation line 204, and transported to a single or a plurality of analyzing units 205 that implement the analysis of the samples. The IDs of the sample racks transported to each analyzing unit and the samples loaded on the sample racks are read by the sample identification ID reader 206 provided for the analyzing unit, and the analyzing unit recognizes the sample rack and the samples to implement analysis needed for the samples. The analyzing unit comprises a reagent disk 208 that houses a plurality of reagents to be used for implementing the analysis. On each reagent necessary for the analysis, an ID 207 for uniquely identifying each reagent is affixed, and the reagent is installed on the reagent disk by the user in advance of the analysis. Further, the analyzing unit comprises a reagent ID reader 209 that identifies each reagent installed on the reagent disk. A sample rack on which necessary analysis has been implemented is transported to a sample housing unit 210. Each of the sample loading unit, the analyzing unit, and the sample housing unit is connected to the operating unit PC 212 of the automatic analyzing device via a hub 211 and through a network cable. The operating unit PC is connected to a higher-order host system via a network. The user requests for analysis (request for measurement) of each sample from the higher-order host system or the operating unit PC, load the samples to be analyzed on the sample rack and places it on the sample loading unit. The information for which the analysis is requested is transmitted to the analyzing unit via a network, and the result of analysis at the analyzing unit is transmitted to the operating unit PC and the higher-order host system. Further, the operating unit PC is connected to a remote system 216 via a public line. The remote system accumulates information including analysis parameter that represents analysis condition information in which the reagent for use on the automatic analyzing device is used, and distributes the analysis parameter according to the request from the operating unit PC. The distributed analysis parameter is stored in the operating unit PC, and transmitted to the analyzing unit upon the analysis of the samples. Further, the operating unit PC comprises an identification ID reader 213 that can read sample ID, a sample rack ID and a reagent ID. The user uses the identification ID reader as an input device for inputting a key (ID) for searching for the test information the user wishes to refer to based on the sample ID, sample rack ID and reagent ID. In general, the sample ID, the sample rack ID and the reagents ID used in the present automatic analyzing device are implemented as, specifically, a bar code or an RFID or the like. Further, identification ID reader that reads each ID corresponds to this. Further, the identification ID reader to be provided on the operating unit PC is assumed to be used in two ways, including one case where an examination engineer, the supposed user, holds by one hand a sample or a reagent to which the identification ID that is the reading target is affixed, or in the other case where the examination engineer holds them by both hands. Therefore, it is preferable for the identification ID reader to have a handy shape and have an identification ID reader holder stand 217 and be of a shape fixable on a desk or the like. FIG. 1 is a diagram showing the relationship between a work flow for referring to test information and screen switching on the operating unit PC in the case where the automatic analyzing device of the present invention is used. Pressing, by the user 101, of the search button displayed on each screen 102 displayed on the operating unit PC104 or pressing, by the user 101, of the searching switch provided on the identification ID reader 105 provided on the operating unit PC invokes displaying of the search menu screen 103 on the operating unit PC. Here, considering that keyboard or mouse operations may be bothersome when the examination engineer, the supposed user, wears gloves, the monitor screen of the operating unit PC desirably has a touch panel function. Next, the user selects their desired searching method on the search menu screen, and reads, by the identification ID reader, any of IDs of the sample ID 106, the sample rack ID 107 and the reagents ID 108 that is their desired target of the test information reference. The automatic analyzing device searches for the test information that matches the ID read as a key in the searching process 109. Next, the automatic analyzing device displays the retrieved test information on the search result screen 110. When the user selects the target test information from the search result screen, a target detailed screen 111 that details the selected test information is displayed. When in the searching process 109 the matching test information includes only single item, the search result screen is not displayed and the target detailed screen is directly displayed. By providing each screen with a back button to go back to the preceding screen, and allowing freely switching between the searching screen and each screen, it becomes to eliminate the necessity to again perform searching and to thereby improve the operability. Further, a work flow is possible in which the operation is initiated from the reading of the identification ID without displaying the search menu screen, by adopting a configuration in which the identification ID reader can always perform reading. By being provided with the above-described information reference function, the device allows referencing to the target test information with easy operations and without understanding a complex structure of the screen.

As an application of the technique of the present invention, it is possible to provide means for reading the above-described identification ID and means for searching for the identification ID at the higher-order host system 215 or a remote system 216 connected to the automatic analyzing device via a network and shown in FIG. 2.

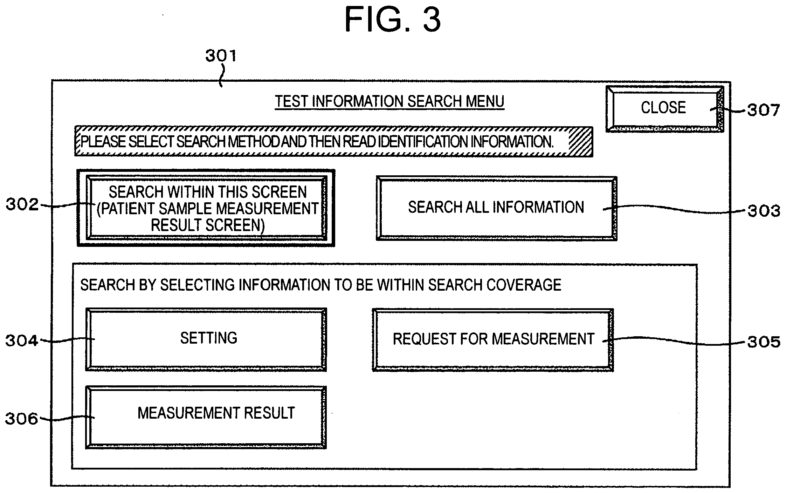

FIG. 3 shows one example of the search menu screen. On the search menu screen 301, the test information that the user wishes to refer to is selected. The in-the-screen search button 302 is a button to be selected when the user wishes to perform searching in test information displayed on the screen from which the search menu screen is activated. It is preferable that, the title of a screen is displayed on the in-the-screen search button, or the search menu screen is displayed as a dialog screen or so, so that the screen in the background from which the search menu screen is activated can be recognized. An all test information item search button 303 is a button to be selected when the user wishes to search all the items of test information stored in the system. A setting information search button 304, a measurement request information search button 305, a measurement result information search button 306 are buttons to be selected when searching of each item of the test information is desired. Other than the above, any buttons for any search coverage that the users may possibly wish to search may be added. Since it is assumed that, from the operative standpoint or the standpoint of specifying the information, there is high possibility that the in-the-screen search button is used, it is preferable that the default state at the time of displaying the search menu screen is such that the in-the-screen search button is selected to thereby allow transition to ID-reading operation without any operation on the search menu screen. Further, preferably, configuring user setting may be allowed to determine which one of the buttons is to be the default button. A close button 307 is a button that, when pressed, causes the search menu screen to be closed to switch back to the screen from which the search menu scree is activated.

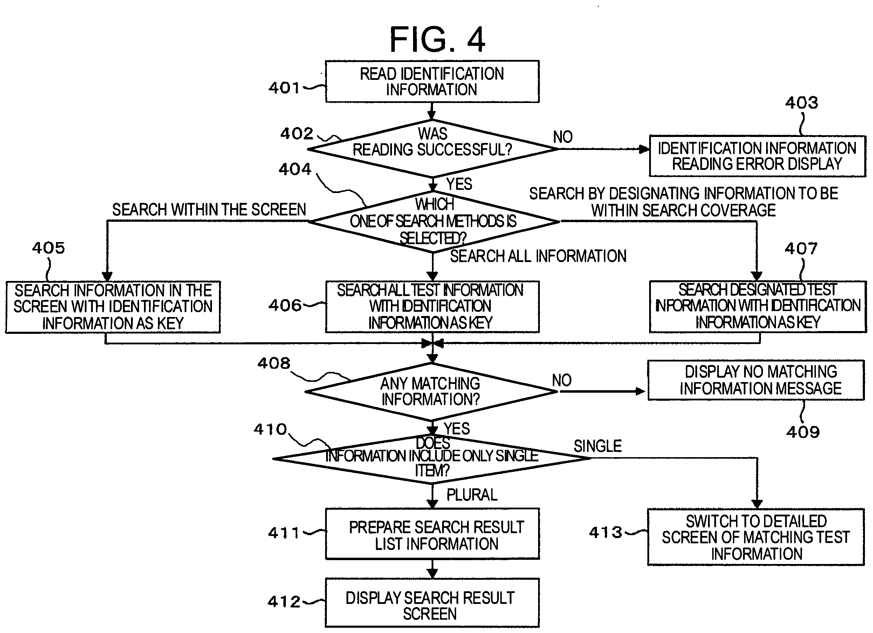

FIG. 4 is a flowchart of a test information searching process. In step 401, when the identification ID is read, it is determined in step 402 whether the reading was successful. If the reading was unsuccessful, identification information reading error is displayed in step 403. If the reading was successful, the searching method selected by the user in step 404 in the search menu screen is determined. If the searching method is for searching within the screen, searching process is performed with the identification ID read in step 405 as a key on the test information displayed on a screen on which the search menu screen is activated. Further, when the searching method is searching all the items of information, searching process is implemented on all the items of test information with the identification ID read in step 406 as a key. Further, when the searching method is searching by designating the target information, searching process is performed on the designated test information with the identification ID read in step 407 as a key. It is determined in step 408 whether the matching test information exists as a result of searching process in step 405 or 406 or 407. When there is no matching test information, a message indicating that there is no matching information is displayed in step 409. When there is matching test information, it is determined in step 410 whether the matching test information includes single item or a plurality of items. When the test information includes a plurality of items, search result listing information to be displayed on the search result screen is prepared in step 411, and the search result screen is displayed in step 412. When in step 410 the matching test information includes only a single item, the detailed screen of the matching test information is displayed in step 413 directly.

A searching method is conceivable in which, by consecutively reading sample IDs or the reagent IDs, searching is performed with the read plurality of identification IDs are used as a compound key. Further, by further providing means for designating logical conditions expressed by AND or OR in the screen, it becomes possible to perform various searching. For example, by searching a sample ID and a reagent ID with AND condition, it is possible to search for a measurement result in which the reagent is used on the sample.

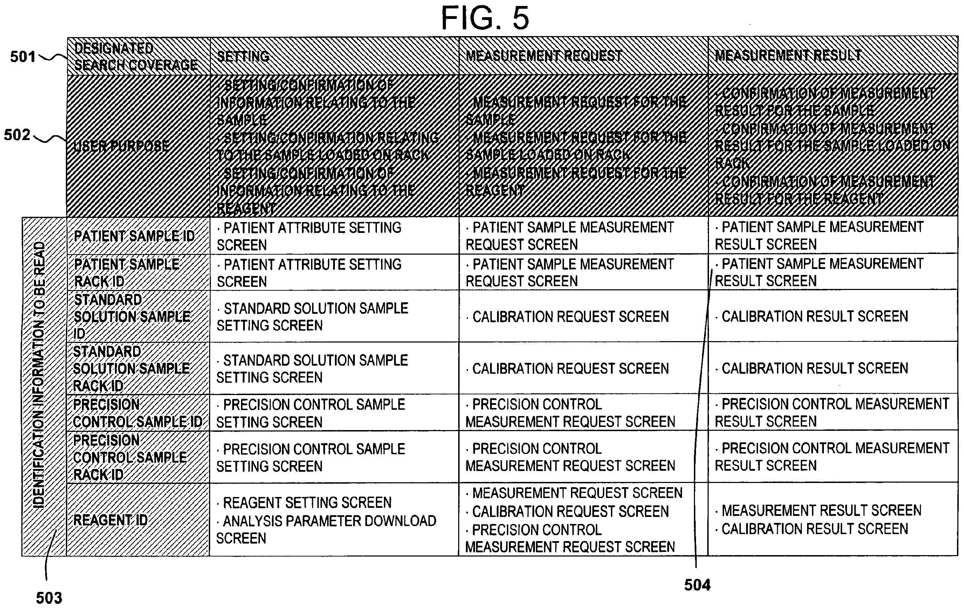

FIG. 5 shows a matrix for determining a screen to be within a specific search coverage when designated test information is searched for with the identification information read in step 407 in FIG. 4 as a key. A designated search coverage 501 includes a search coverage that the user selected with a button in the search menu screen shown in FIG. 3. A user's purpose 502 includes a possible purpose of a user having selected 501. Identification information to be read 503 is each identification ID that the user reads with the identification ID reader after selection of 501. In general, samples to be an analysis target of the automatic analyzing device includes patient samples collected from patients, standard solution samples for calibration of device, and accuracy control samples for accuracy control of the device. Further, there individually exists a dedicated sample rack for loading each sample. When, for example, the user selects measurement result on a search menu screen and successively causes the identification ID reader to read the patient sample rack ID, the device determines, according to the present matrix, that the confirmation of result of measurement for the concerned samples loaded on the rack is the user's purpose, and performs a process with the test information displayed on the patient sample measurement result screen 504, which displays a result of analysis of the patient sample, as a search coverage. By using such a matrix, it becomes possible, by merely performing simple decision making and identification ID reading, to eliminate necessity of understanding structures of complex and many screens of the automatic analyzing device and the accompanying operations and input of several tens of digits of identification information, and it becomes possible to easily reference to the target test information from many items of test information. In particular, the device is very effective when night workers or doctors who do not usually use the automatic analyzing device and unfamiliar thereto, in addition to the examination engineers who are the supposed users, make reference to target information.

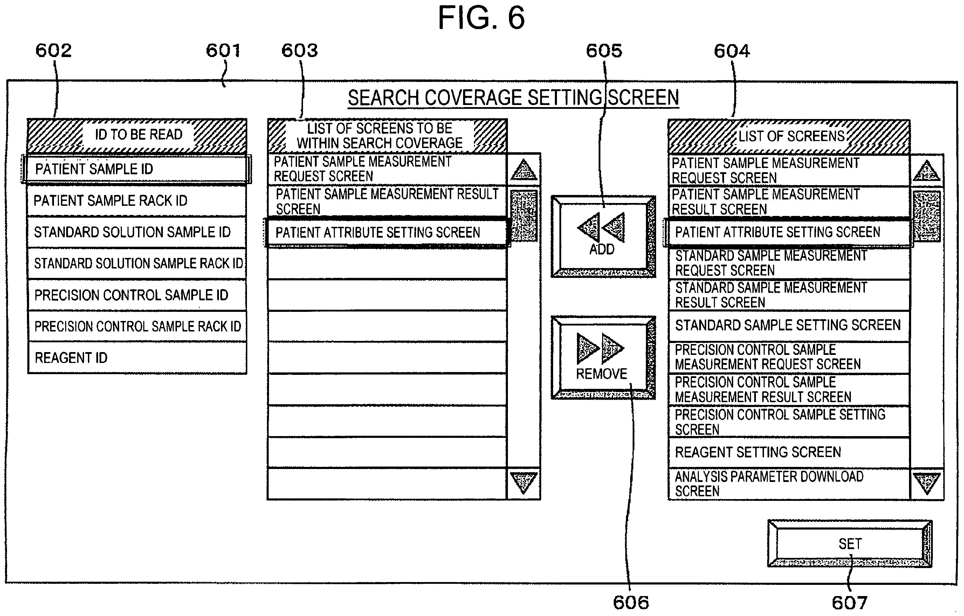

FIG. 6 shows an example of a search coverage setting screen in which a screen to be a search coverage that is set when all the items of test information are searched for with the identification information read in step 406 in FIG. 4 as a key. When all the items of test information are determined as being within the search coverage, there may be many items of matching test information depending on the conditions, and many items of test information that are out of the user's intention are displayed on the search result screen, which might compromise visibility, or causes increase in time for searching test information due to the increased number of parameters. To overcome the problem, the search coverage setting screen 601 may be configured so that the user is allowed to freely set a screen to be within the search coverage and the device narrows down the search coverage based on the setting for each classification of the identification ID to be read, so that appropriate searching as intended by the user can be performed. The reading identification ID list 602 displays a listing of the identification IDs to be the target of reading by the identification ID reader. The search coverage screen listing 603 displays a screen listing that is the search coverage with the identification ID selected in 602. The screen listing 604 displays a screen listing of the automatic analyzing device. First, the user selects an identification ID the user wishes to perform setting therefor in 602. When the identification ID is selected, a listing of screens to be within the search coverage with the identification ID selected in 603 is displayed. Next, the user selects a screen that the user wishes to add as a search coverage from 604 and presses an add button 605. When the add button is pressed, a screen selected in 604 is additionally displayed in 603 as a new search coverage screen. Further, when there is a screen the user wishes to exclude from the search coverage, the user selects it in 603 and presses removal button 606 to thereby erase the display in 603 and exclude from the search coverage. Here, preferably, only those screens which are not within the search coverage are displayed on 604 so that the screen that are within search coverage and those which are not are easily recognized. Finally, the user presses a setting button 607 to thereby set conditions to be within the search coverage. In the part 603, screens to be within the search coverage can be set with the same operation for each identification ID in 602. In the example of display in FIG. 6, the targets of search with the patient sample ID are the following three screens: the patient samples measurement request screen; the patient sample measurement result screen and the patient attribute setting screen.

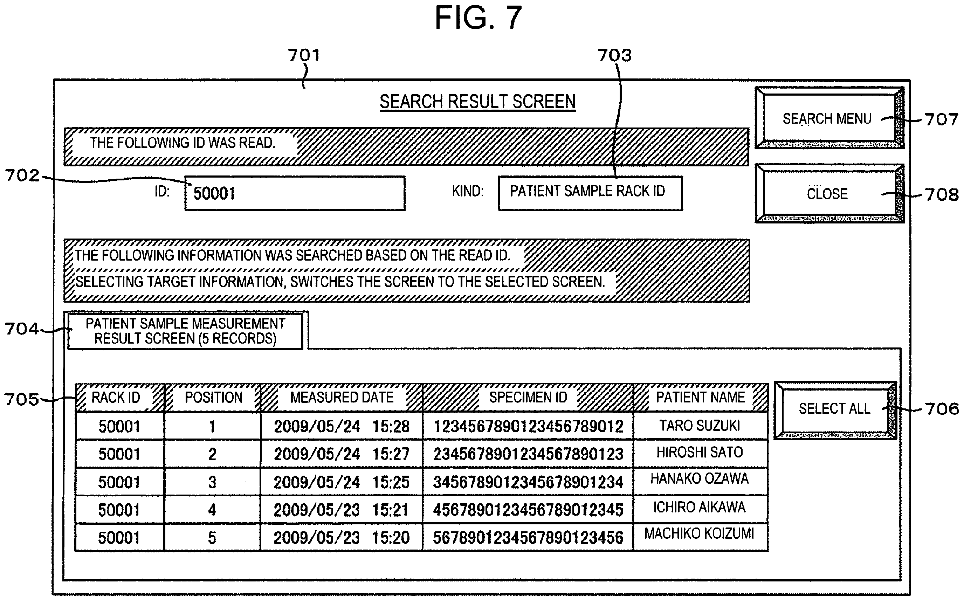

FIG. 7 shows an example of a search result screen to be displayed in a case where there are a plurality of items of matching test information as a result of searching process. In a search result screen 701, an identification information read by the identification ID reader and a listing of the test information retrieved from the identification information are displayed. The read identification ID is displayed in the read identification ID display area 702, and the kind of the identification ID is displayed on the reading identification ID classification display area 703. The retrieved test information is gathered for each screen and displayed as an organized-by-screen search result display tab 704. Therefore, in a case where there are a plurality of matching screens, they are displayed as a plurality of tabs.

In the organized-by-screen search result display tab, the number of records of the matching test information is displayed as auxiliary information. On the organized-by-screen search result display tab, the matching test information in each screen is displayed as search result display list 705 in a listed manner. Since it is very likely that the user intends to do search with the immediately preceding test information as the target, it is preferable that the test information to be displayed is sorted in the descending order of date. Further, it is preferable to implement a function in which pressing of any heading of the list invokes free sorting by the heading in ascending or descending order. The example of display in FIG. 7 shows a case where the patient sample rack ID is read, and measurement results relating to 5 patient samples loaded on the rack are found. The user, when the search result screen is displayed, seeks the target test information and selects it from the organized-by-screen search result display tab and the search result display list. The selection of the test information is performed by directly selecting the display on the search result display list or by the select-all button 706 for selecting all the items of information displayed on the list. When the test information is selected, the screen switches to a detailed screen in which the corresponding test information exists. In a case where another search is newly performed, by navigating back to the search menu screen, the user depresses a search menu button 707, and where the search result screen is to be closed, the user depresses the close button 708. In order that the latest search results are referenced anytime from each screen of the automatic analyzing device, latest search results are stored. Preferably, predetermined number of the performed past search results are stored to allow past search results to be displayed.

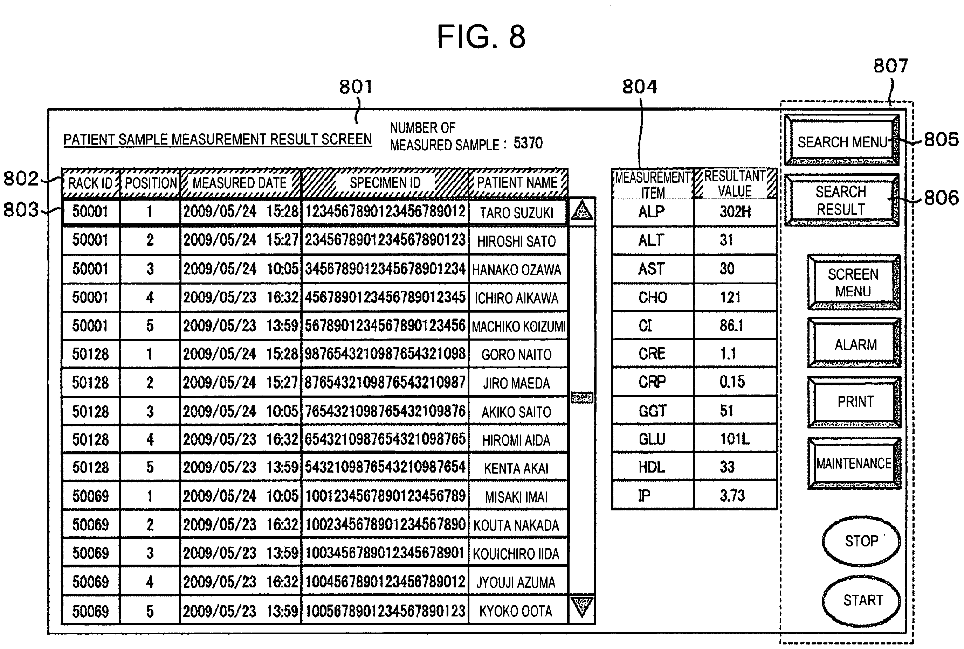

FIG. 8 shows a detailed screen of test information users intends to refer to. This example shows a case where test information in the first line of the search result display list is selected from the search results of FIG. 7. A patient sample measurement result screen 801 in which the selected test information exists is displayed, and test information 802 is displayed with the target test information 802 selected in the search result screen being automatically selected (being in the stage of having the focus). Further, interlocking with this, test information details 804 that is the target is displayed. In the example of FIG. 8, measurement result of test information selected in the search result screen is automatically displayed. It is preferable to perform a display process such that the test information is displayed in the uppermost row of the measurement result display list as shown in 803 in order to improve visibility. A search menu button 805 is a button for displaying the search menu screen. In order that the search menu screen can be displayed from any screen, it is preferable that the search menu button is disposed in a screen-independent display area 807 that is global and does not depend on the display of each screen. The search result button 806 is a button for displaying the search result screen. When the search result button is depressed, a latest search result that is stored is displayed. Similarly to the search menu button, it is preferable that the search result button is disposed on the global screen-independent display area.

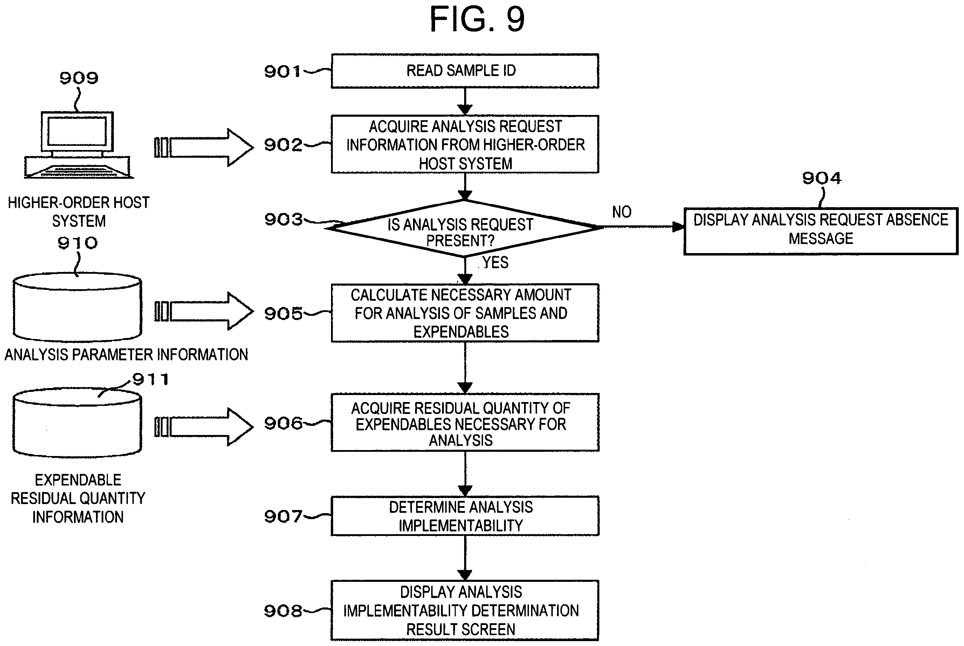

FIG. 9 shows a process flowchart in which information regarding a request for analyzing a sample is acquired from a higher-order host system and it is determined whether the analysis is implementable or not in advance of the analysis by utilizing the present invention. In conventional automatic analyzing devices, a sample ID is read by a sample ID reader 206 that is provided in the device and shown in FIG. 2 by introducing the samples in the device and initiating the analysis, and request information for the analysis of the sample is acquired from the higher-order host system 215. Therefore, there has been no way to know what kind of analysis is requested for the samples in advance of the analysis. Therefore, the users have not been not able to determine whether it is possible to proceed the analysis with the analysis request as-is by referencing to the analysis request in a case where, for example, the analysis target is a specimen from an infant and amount of blood collection is very small, or the samples are in short by consumption in analysis in other systems or being lost erroneously, or where abnormality including sample turbidity or the like is caused in a pre-analysis process, such as, centrifugation. Further, for the same reason, it was not possible to determine in advance of the analysis whether residual quantity of expendables including reagents needed in the analysis and provided in the device is sufficient. As a result of that, if it turns out that the analysis is not implementable, that means that the user have once failed in the analysis and it comes to that the user again attempts the analysis, spending enormous effort. In the backdrop of this, there are possible causes of the problem, as follows: the larger the scale of the examination the user intends to perform becomes, the more likely the higher-order host system and the automatic analyzing device are operated in different rooms in consideration of installation space and a user who requests analysis to the higher-order host system is a different person from a user who implements the analysis by the automatic analyzing device, because of splitting of work responsibility.

In the present invention, in order to overcome the above-described problem, as shown in FIG. 9, when a sample ID is read in step 901, analysis request information regarding the sample ID is acquired in step 902 from a higher-order host system 909 connected to the automatic analyzing device via a network. In step 903, presence or absence of the analysis request information of the sample ID is determined, and if the analysis request is not present, analysis request absence message is displayed in step 904. If an analysis request is present, the amount of samples and expendables needed in the analysis request acquired in step 902 is calculated based on the analysis parameter information 910 stored in the automatic analyzing device in step 905. Next, in step 906, expendable residual quantity information 911 stored in the automatic analyzing device regarding the expendables to be needed in the analysis request acquired in step 902 is acquired. In step 907, the amount of expendables necessary for the analysis request calculated in step 905 is compared with the residual quantity of the expendables acquired in step 906 to determine whether the analysis can be performed. In step 908, the screen displays thereon analysis request information of the sample ID, necessary amount of samples and expendables calculated in step 905, residual quantity of expendables acquired in step 906, result of determination in step 907 on whether analysis can be performed.

By performing such a process in the automatic analyzing device, the user can confirm whether the analysis to be implemented is implementable by merely reading in advance the IDs of samples without initiating the analysis. Thus it becomes possible to do preparation including recollecting samples, reviewing the analysis request, and replenishment of expendables in advance, and prevent failure in analysis caused by sample shortage, sample abnormality and expendable shortage. Further, although in FIG. 9 a basic process for a single sample ID is described, it is possible to further improve user work efficiency by reading in step 901 a plurality of sample IDs in a batch, repeating a processes of step 902 to step 907 for the plurality of sample IDs and displaying information regarding the plurality of sample ID on the screen in step 908.

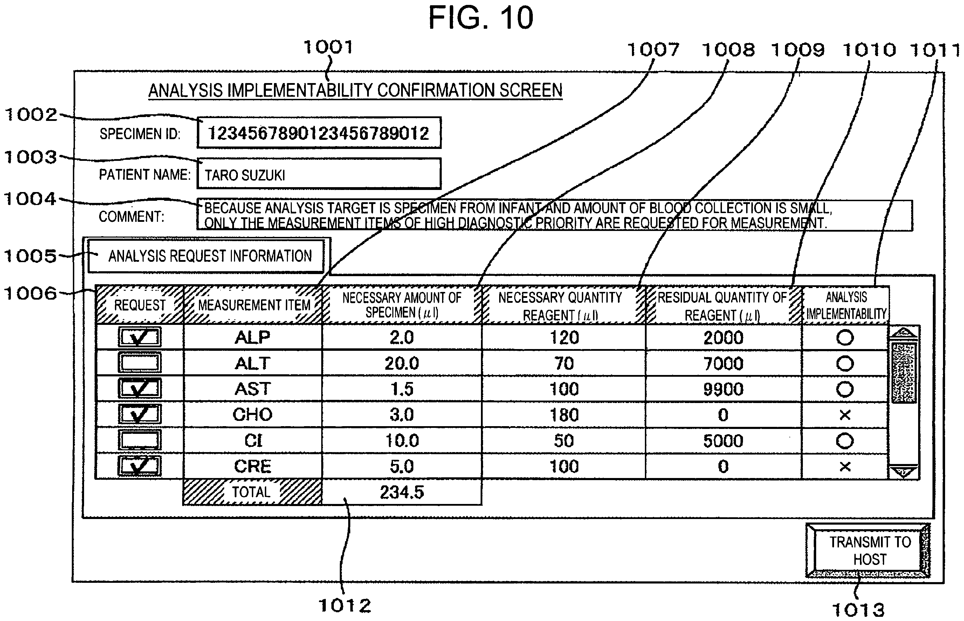

FIG. 10 shows a specific example of the analysis implementability confirmation screen to be displayed in step 908 in FIG. 9. The analysis implementability confirmation screen 1001 displays analysis request information acquired from the higher-order host system regarding the sample ID read by the user and a listing of analysis implementability determination result. Further, the device has a function for a user to review an analysis request and transmit the changed analysis request to the higher-order host system. In a specimen ID display area 1002, the read sample ID is displayed. The patient name display area 1002 displays a patient name based on the analysis request information acquired regarding the sample ID from the higher-order host system. Further, in an analysis request information display area 1005, analysis request information acquired from the higher-order host system is displayed. In a measurement item 1007, measurement item requested for the sample is displayed. Further, for each requested measurement item, necessary amount of samples acquired from the analysis parameter information 910 in FIG. 9 is displayed in a specimen necessary amount display area 1008, and the necessary amount of reagent is displayed in a reagent necessary amount 1009. In the reagent residual quantity display area 1010, residual quantity of a reagent for each measurement item acquired from the expendable residual quantity information 911 in FIG. 9 is displayed. In the analysis implementability display area 1011, the result of determination on whether the analysis is implementable is displayed based on the necessary amount reagents and the residual quantity. In the total necessary specimen amount display area, the total amount of specimen necessary for all the measurement items requested for the samples is displayed. The user of the automatic analyzing device confirms the displayed measurement item, the necessary amounts of specimens, necessary amount and residual quantity of reagents and analysis implementability, and determines whether the analysis can be performed for the content requested from the higher-order host system based on the amount and the state of the actual samples, and the residual quantity of reagents. Consequently, samples and reagents in short are replenished. Further, when it is not possible to perform replenishment, an analysis request setting check box 1006 is provided in order to make the requested analysis content changeable. For example, for a reason that the sample is a specimen from an infant and the amount of collected blood is small and it is not possible to perform analysis for all the measurement items requested from a higher-order host system, setting is configured to exclude measurement items of low diagnostic priority on the analysis request so that only the measurement items of high diagnostic priority are subject to measurement. The default state of the display of the analysis request setting check box 1006 is such that all the items are set to be measured. Further, a comment input area 1004 is provided so that the user can input the reason why the analysis request setting is changed. Furthermore, a host transmission button 1013 transmitting changed analysis request information in the automatic analyzing device to a higher-order host system and update the changed analysis request information in the higher-order host system is provided. This makes it possible to ensure the consistency between the analysis request information of the samples stored in the automatic analyzing device and the higher-order host system. As described in the explanation for FIG. 9, it is possible to further improve the work efficiency of the user by reading a plurality of sample IDs in a batch and displaying the information for the plurality of sample IDs on the screen.

### Reference Signs List

101, 214 user
102 each screen
103 search menu screen
104, 212 operating unit PC
105, 213 identification ID reader
106, 201 sample ID
107, 202 sample rack ID
108, 207 reagent ID
109 searching process
110, 701 search result screen
111 target detailed screen
203 sample introduction unit
204 transportation line
205 analyzing unit
206 sample ID reader
208 reagent disk
209 reagent ID reader
210 sample housing unit
211 hub
215, 909 higher-order host system
216 remote system
217 identification ID reader holder stand
301 test information search menu screen
302 in-the-screen search button
303 all screen search button
304 setting information search button
305 measurement request information search button
306 measurement result information search button
307, 708 close button
401-413, 901-908 flowchart step
501 designated search coverage
502 user purpose
503 identification information to be read
504, 801 patient sample measurement result screen
601 search coverage setting screen
602 reading identification ID list
603 within-search-coverage screen listing
604 screen listing
605 add button
606 removal button
607 setting button
702 read identification ID display area
703 reading identification ID classification display area
704 organized-by-screen search result display tab
705 search result display list
706 select all button
707, 805 search menu button
802 test information
803 target test information
804 target test information details
806 search result button
807 screen-independent display area
910 analysis parameter information
911 expendable residual quantity information
1001 analysis implementability confirmation screen
1002 specimen ID display area
1003 patient name display area
1004 comment input area
1005 analysis request information display area
1006 analysis request setting check box
1007 measurement item display area
1008 specimen necessary amount display area
1009 reagent necessary amount display area
1010 reagent residual quantity display area
1011 analysis implementability display area
1012 total necessary specimen amount display area
1013 host transmission button

## Claims

1. An automatic analyzing device comprising:
an information recording medium (106-108; 201, 202, 207) provided on a container for containing a sample or a reagent, or a receiver on which the container is loaded, and having identification information recorded therein for identifying the sample or the reagent to be contained;
reading means (105; 206, 209, 213) for reading identification information recorded in the information recording medium (106-108; 201, 202, 207);
storage means for storing information related to an analysis in association with the identification information;
searching means for, in response to an event in which the identification information recorded in the information recording medium (106-108; 201, 202, 207) is read by the reading means (105; 206, 209, 213), searching for information relating to the analysis associated with the identification information read by the reading means (105; 206, 209, 213) from among the information stored in the storage means;
display means (701) for displaying information relating to the analysis searched for by the searching means; and
communication means (211) for connecting to a higher-order system (214; 909) storing information that relates to a request for analysis of a sample that is within a search coverage, wherein the display means (701) is adapted to display information acquired in advance of the analysis through the communication means (211) from the higher-order system (214; 909),
**characterised by** further comprising:
determination means for determining analysis implementability based on a request for analysis of the samples acquired from the higher-order system (214; 909) and the information of an expendable loaded on the automatic analyzing device and needed for the analysis, wherein the display means (701) is adapted to display a determination result based on the determination means, and the display means (701) comprises means (1006) for allowing a user to change the analysis request information for a sample; and
instruction means that is adapted to transmit the changed analysis request information to the higher-order system (214; 909), and instruct the higher-order system (214; 909) to update the stored analysis request information.

2. The automatic analyzing device according to claim 1, further comprising:
selection means (706) for selecting any of information relating to the analysis displayed on the display means (701); and
control means for, when a selection is made by the selection means (706), controlling the display means (701) to switch a display screen (110) to an original screen (111) that displays information relating to the selected analysis.

3. The automatic analyzing device according to claim 2, further comprising switching means for switching to a display screen (110) displaying the information relating to the analysis searched for by the searching means.

4. The automatic analyzing device according to any one of claims 1 to 3, wherein the searching means comprises designating means (304-306) for designating information relating to the analysis that is within a search coverage.

5. The automatic analyzing device according to any one of claims 1 to 3, wherein an invocation button (805) for invoking a searching screen (301) of the searching means is displayed on a common display area (807) independent from the display screen (801).

6. The automatic analyzing device according to any one of claims 1 to 3, wherein an invocation switch for invoking the searching screen (301) of the searching means is provided on the identification information reading means (105; 206, 209, 213).

7. The automatic analyzing device according to claim 1, wherein the identification information reading means (105; 206, 209, 213) or the search result display means (701) is provided on an operating unit (104) controlling a single or a plurality of analyzing units (205) that implement analysis.

## Patentansprüche

1. Automatisches Analysegerät mit
einem Informationsaufzeichnungsmedium (106-108; 201, 202, 207), das auf einem Behälter zum Aufbewahren einer Probe oder eines Reagens, oder einer Aufnahme, auf der der Behälter angeordnet ist, vorgesehen ist und das eine darin gespeicherte Identifikationsinformation zum Identifizieren der Probe oder des Reagens aufweist, das darin enthalten sein soll,
einer Leseeinrichtung (105; 206, 209, 213) zum Lesen der in dem Informationsaufzeichnungsmedium (106-108; 201, 202, 207) aufgezeichneten Identifikationsinformation,
einer Speichereinrichtung zum Speichern von Information bezüglich einer Analyse in Zusammenhang mit der Identifikationsinformation,
einer Sucheinrichtung, um, bei Auftreten eines Ereignisses, in dem die in dem Informationsaufzeichnungsmedium (106-108; 201, 202, 207) aufgezeichnete Identifikationsinformation durch die Leseeinrichtung (105; 206, 209, 213) ausgelesen wird, nach Information unter der in der Speichereinrichtung gespeicherten Information zu suchen, die sich auf die Analyse in Zusammenhang mit der durch die Leseeinrichtung (105; 206, 209, 213) ausgelesene Identifikationsinformation bezieht,
einer Anzeigeeinrichtung (701) zum Anzeigen von Information bezüglich der durch die Sucheinrichtung gesuchten Analyse, und
einer Kommunikationseinrichtung (211) zum Anschließen eines Systems höherer Ordnung (214; 909), das Information bezüglich einer Anfrage für eine Probenanalyse, die innerhalb eines Suchbereichs liegt, speichert, wobei die Anzeigeeinrichtung (701) dazu ausgelegt ist, Information anzuzeigen, die vor der Analyse durch die Kommunikationseinrichtung (211) von dem System höherer Ordnung (214; 909) erhalten wurde,
**gekennzeichnet durch**
eine Bestimmungseinrichtung zum Bestimmen der Analyseimplementierbarkeit basierend auf einer Anfrage zur Analyse der Proben, die von dem System höherer Ordnung (214; 909) empfangen wurde und der Information eines Verbrauchsmaterials, das auf dem automatischen Analysegerät angeordnet ist und für die Analyse benötigt wird, wobei die Anzeigeeinrichtung (701) dazu ausgelegt ist, ein auf der Bestimmungseinrichtung basierendes Bestimmungsergebnis anzuzeigen, und die Anzeigeeinrichtung (701) eine Einrichtung (1006) aufweist, um einem Benutzer eine Änderung der Analyseanfrageinformation für eine Probe zu erlauben, und
eine Befehlseinrichtung, die dazu ausgelegt ist, die geänderte Analyseanfrageinformation an das System höherer Ordnung (214; 909) zu senden und dem System höherer Ordnung (214; 909) zu befehlen, die gespeicherte Analyseanfrageinformation zu aktualisieren.

2. Automatisches Analysegerät nach Anspruch 1, ferner mit
einer Auswähleinrichtung (706) zum Auswählen einer beliebigen Information bezüglich der Analyse, die auf der Anzeigeeinrichtung (701) angezeigt wird, und
einer Steuereinrichtung, um, wenn eine Auswahl durch die Auswähleinrichtung (706) vorgenommen wurde, die Anzeigeeinrichtung (701) dazu zu steuern, einen Anzeigebildschirm (110) auf einen ursprünglichen Bildschirm (111) umzuschalten, der Information bezüglich der ausgewählten Analyse anzeigt.

3. Automatisches Analysegerät nach Anspruch 2, ferner mit einer Umschalteinrichtung zum Umschalten auf einen Anzeigebildschirm (110), der Information bezüglich der durch die Sucheinrichtung gesuchten Analyse anzeigt.

4. Automatisches Analysegerät nach einem der Ansprüche 1 bis 3, wobei die Sucheinrichtung eine Kennzeichnungseinrichtung (304-306) zum Kennzeichnen von Information bezüglich der Analyse aufweist, die innerhalb eines Suchbereichs liegt.

5. Automatisches Analysegerät nach einem der Ansprüche 1 bis 3, wobei eine Aufruftaste (805) zum Aufrufen eines Suchbildschirms (301) der Sucheinrichtung auf einer allgemeinen Anzeigefläche (807) unabhängig von dem Anzeigebildschirm (801) angezeigt wird.

6. Automatisches Analysegerät nach einem der Ansprüche 1 bis 3, wobei ein Aufrufschalter zum Aufrufen des Suchbildschirms (301) der Sucheinrichtung in der Identifikationsinformations-Leseeinrichtung (105; 206, 209, 213) vorgesehen ist.

7. Automatisches Analysegerät nach Anspruch 1, wobei die Identifikationsinformations-Leseeinrichtung (105; 206, 209, 213) oder die Suchergebnis-Anzeigeeinrichtung (701) auf einer Betriebseinheit (104) vorgesehen ist, die eine einzelne oder mehrere Analyseeinheiten (205) steuert, die die Analyse implementieren.

## Revendications

1. Dispositif d'analyse automatique comprenant :
un support d'enregistrement d'informations (106-108 ; 201, 202, 207) prévu sur un récipient pour contenir un échantillon ou un réactif, ou un récepteur sur lequel le récipient est chargé, et ayant des informations d'identification enregistrées sur celui-ci pour identifier l'échantillon ou le réactif destiné à être contenu ;
un moyen de lecture (105 ; 206, 209, 213) pour lire des informations d'identification enregistrées dans le support d'enregistrement d'informations (106-108 ; 201, 202, 207) ;
un moyen de stockage pour stocker des informations se rapportant à une analyse en association avec les informations d'identification ;
un moyen de recherche pour, en réponse à un événement dans lequel les informations d'identification enregistrées dans le support d'enregistrement d'informations (106-108 ; 201, 202, 207) sont lues par le moyen de lecture (105 ; 206, 209, 213), rechercher des informations se rapportant à l'analyse associée avec les informations d'identification lues par le moyen de lecture (105 ; 206, 209, 213) parmi les informations stockées dans le moyen de stockage ;
un moyen d'affichage (701) pour afficher des informations se rapportant à l'analyse recherchée par le moyen de recherche ; et
un moyen de communication (211) pour une connexion à un système d'ordre supérieur (214 ; 909) stockant des informations qui se rapportent à une demande d'analyse d'un échantillon qui est à l'intérieur d'une couverture de recherche, dans lequel le moyen d'affichage (701) est adapté à afficher des informations acquises au préalable de l'analyse par l'intermédiaire du moyen de communication (211) auprès du système d'ordre supérieur (214 ; 909),
**caractérisé en ce que** comprenant en outre :
un moyen de détermination pour déterminer une capacité de mise en oeuvre d'analyse sur la base d'une demande d'analyse des échantillons acquise auprès du système d'ordre supérieur (214 ; 909) et des informations d'un consommable chargé sur le dispositif d'analyse automatique et nécessaire pour l'analyse, dans lequel le moyen d'affichage (701) est adapté à afficher un résultat de détermination sur la base du moyen de détermination, et le moyen d'affichage (701) comprend un moyen (1006) pour permettre à un utilisateur de changer les informations de demande d'analyse pour un échantillon ; et
un moyen d'instruction qui est adapté à transmettre les informations de demande d'analyse changées au système d'ordre supérieur (214 ; 909), et ordonner au système d'ordre supérieur (214 ; 909) de mettre à jour les informations de demande d'analyse stockées.

2. Dispositif d'analyse automatique selon la revendication 1, comprenant en outre :
un moyen de sélection (706) pour sélectionner l'une quelconque d'informations se rapportant à l'analyse affichées sur le moyen d'affichage (701) ; et
un moyen de commande pour, lorsqu'une sélection est effectuée par le moyen de sélection (706), commander le moyen d'affichage (701) pour commuter un écran d'affichage (110) sur un écran d'origine (111) qui affiche des informations se rapportant à l'analyse sélectionnée.

3. Dispositif d'analyse automatique selon la revendication 2, comprenant en outre un moyen de commutation pour commuter sur un écran d'affichage (110) affichant les informations se rapportant à l'analyse recherchées par le moyen de recherche.

4. Dispositif d'analyse automatique selon l'une quelconque des revendications 1 à 3, dans lequel le moyen de recherche comprend un moyen de désignation (304-306) pour désigner des informations se rapportant à l'analyse qui est à l'intérieur d'une couverture de recherche.

5. Dispositif d'analyse automatique selon l'une quelconque des revendications 1 à 3, dans lequel un bouton d'invocation (805) pour invoquer un écran de recherche (301) du moyen de recherche est affiché sur une zone d'affichage commune (807) indépendante de l'écran d'affichage (801).

6. Dispositif d'analyse automatique selon l'une quelconque des revendications 1 à 3, dans lequel un commutateur d'invocation pour invoquer l'écran de recherche (301) du moyen de recherche est prévu sur le moyen de lecture (105 ; 206, 209, 213) d'informations d'identification.

7. Dispositif d'analyse automatique selon la revendication 1, dans lequel le moyen de lecture (105 ; 206, 209, 213) d'informations d'identification ou le moyen d'affichage (701) de résultat de recherche est prévu sur une unité opérationnelle (104) commandant une ou une pluralité d'unités d'analyse (205) qui mettent en oeuvre une analyse.
